# EUROPEAN PATENT SPECIFICATION

(11) **EP 3 485 733 B1**
(45) Date of publication and mention of the grant of the patent: **20.09.2023**
(21) Application number: 17827887.5
(22) Date of filing: 07.07.2017
(51) Int. Cl.: A01N 25/04, C12N 1/20, A01G 7/06, A01N 63/22, A01P 21/00

(54) **METHOD FOR PROMOTING GROWTH OF PLANTS BY USING BACILLUS AMYLOLIQUEFACIENS**
VERFAHREN ZUR FÖRDERUNG DES WACHSTUMS VON PFLANZEN DURCH VERWENDUNG VON BACILLUS AMYLOLIQUEFACIENS
PROCEDE POUR FAVORISER LA CROISSANCE DE PLANTES A L'AIDE DE BACILLUS AMYLOLIQUEFACIENS

(30) Priority: 12.07.2016 KR 20160088234
(43) Date of publication of application: 22.05.2019
(73) Proprietor: GLOBAL AGRO.,LTD., Seoul, 06627 (KR)
(72) Inventor: KIM, Joung Woong, Gyeongju-si Gyeongsangbuk-do 35058 (KR); HONG, Ki Chang, Gyeongju-si Gyeongsangbuk-do 38108 (KR); CHO, Hyun Jong, Gyeongju-si Gyeongsangbuk-do 38051 (KR); KIM, Beom Seok, Seoul 04346 (KR)
(74) Representative: Tiburzi, Andrea
(86) International application number: PCT/KR2017/007321
(87) International publication number: WO 2018/012815

(56) References cited:
- WO-A1-2013/165607
- KR-A- 20060 021 162
- KR-A- 20120 004 856
- KR-A- 20130 008 673
- KR-A- 20130 008 673
- KR-A- 20150 119 601
- KR-A- 20160 059 296
- JEONG DO KIM ET AL: "Evaluation of the endophytic nature of Bacillus amyloliquefaciens strain GYL4 and its efficacy in the control of anthracnose : Endophytic biocontrol agent for anthracnose management", PEST MANAGEMENT SCIENCE, vol. 72, no. 8, 1 December 2015 (2015-12-01), pages 1529-1536, XP55664951, BOGNOR REGIS; GB ISSN: 1526-498X, DOI: 10.1002/ps.4181
- QIAO, JUN-QING et al.: "Stimulation of Plant Growth and Biocontrol by Bacillus Amyloliquefaciens Subsp. Plantarum FZB42 Engineered for Improved Action", Chemical and Biological Technologies in Agriculture, vol. 1, no. 12, 2014, pages 1-14, XP021219993,

## Description

### Technical Field

The present invention relates to a method of promoting growth of a crop, comprising:mixing a liquid-phase growth-promoting agent for a crop with water; and applying a mixture thus obtained to the crop;wherein said liquid-phase growth-promoting agent comprises a living bacterium of *Bacillus amyloliquefaciens* GYL4 (accession number KACC91655P) as an effective component.

### Background Art

In general, plants grow naturally while being affected by multiple factors such as climatic conditions, soil characteristics, living organisms, and other environmental factors. Various studies have been conducted into the factors as well as interactions among the multiple factors in order to improve the yield of plants, especially agricultural products.

In recent years, the excessive use of synthetic chemicals in agriculture has resulted in serious problems involving the pollution of agro-ecosystem and agricultural products. In particular, with increasing interest in the safety of agricultural products, agents and methods for promoting the growth of plants, capable of improving the yield of environmentally friendly crops, have become increasingly popular, while numerous studies have been conducted on organic farming materials employing microorganisms as alternatives to synthetic chemicals.

Organic farming is an alternative agricultural production system that guarantees optimal, maximum yield gains while sustaining soil fertility by means of rotated cropping with cereals in forage pasture, crop rotation in which different types of crops are cultivated in the same field year by year in a planned sequence, green manure, which utilizes the stems and leaves of plants as an organic fertilizer source, composting using straw or fallen leaves, and the use of microorganisms for the control of plant diseases.

In organic farming, microorganisms are typically employed to protect plants from pathogens, mainly as antibacterial agents. The most commonly used microbe is *Bacillus subtilis,* and fungi or viruses have also been actively studied. Thus, it has been estimated that the era of so-called microbial pesticides has begun.

In this regard, a study with the bacterium *Bacillus amyloliquefaciens* reported the impact of a *Bacillus* species, *B. amyloliquefaciens* FZB42, on the rhizosphere environment (Soumitra Paul Chowdhury et al., Effects of Bacillus amyloliquefaciens FZB42 on Lettuce Growth and Health under Pathogen Pressure and its Impact on the Rhizosphere Bacterial Community, PLOS ONE Vol. 8 No.7: 1932-6203). Another study investigated the efficacy of *Bacillus amyloliquefaciens* GYL4 on anthracnose (Kim JD et al., Evaluation of the endophytic nature of Bacillus amyloliquefaciens strain GYL4 and its efficacy in the control of anthracnose, Pest Management Sci. 2016. 8; 72(8): 1529-36) .

In addition, Korean Patent Application Publication No. 2013-0008673 discloses a composition for preventing plant anthracnose comprising *Bacillus amyloliquefaciens* GYL4 having antifungal activity against plant pathogenic fungi. Korean Patent Application Publication No. 2008-0095652 discloses a preventive composition having an effect of protecting against plant pathogenic bacteria and fungi comprising *Bacillus amyloliquefaciens* A-7. Korean Patent Application Publication No. 2013-0005592 discloses a composition for protecting against plant pathogenic bacteria and fungi comprising *Bacillus amyloliquefaciens* EML-JUN11. Korean Patent Application Publication No. 2016-0059296 discloses *Bacillus amyloliquefaciens* Ba3, which has antifungal activity against plant pathogens. Korean Patent Application Publication No. 2008-0095652 discloses a *Bacillus amyloliquefaciens* strain A-7, which has an effect of protecting against plant pathogenic bacteria and fungi. Korean Patent Application Publication No. 2014-0010639 describes that *Bacillus amyloliquefaciens* EML-CAP3 produces a peptide having an antibacterial or antifungal effect. Also, with regard to the antibacterial effect of *Bacillus amyloliquefaciens,* a patent document (Korean Patent Registration No. 1390457) describes that *Bacillus amyloliquefaciens* EBN-TK3 has an effect of protecting against a swine-atrophic-rhinitis-causing bacterium and a poultry-typhoid-causing bacterium in the swine and poultry industries. In addition, a composition containing *Bacillus amyloliquefaciens* BAC03 and its use for promoting growth of radish, pepper, beet, carrot, turnip, cucumber and tomato are also known (WO2013/165607). Korean Patent Application Publication No. 2015-0119601 describes a composition containing *Bacillus amyloliquefaciens* KB3 and its use for promoting growth of grass.

As described above, conventional studies associated with the usefulness of *Bacillus amyloliquefaciens* have focused on antibacterial and disease-preventing effects.

In organic farming, organic fertilizers such as green manure and compost nourish the soil and thus stimulate the growth of plants. The term "growth", as used herein, is understood to mean the growth and development of plants, especially agricultural crops, and refers to the process of reaching the stage of maturity, that is, the harvest stage.

Meanwhile, in organic farming, the term "antibacterial and antifungal effect" refers to the inhibition and elimination of plant pathogens, which adversely affect the yield and quality of plants, especially crops. *Bacillus* species are known to secrete about 66 types of antibacterial or antifungal peptides (e.g., bacillomycin and mycobacillin). Of these, compounds such as surfactin, iturin and fengycin are known to inhibit soil pathogens through rhizosphere colonization and antagonistic activity and thus lead to disease resistance of crops, eventually inhibiting pathogen infection and spread. In this way, the term "antimicrobial effect" is distinguished from the term "growth".

Meanwhile, the term "rhizosphere" refers to the environment that surrounds plant roots during plant growth and has a great influence on the health and productivity of plants. In the rhizosphere, various soil microorganisms, such as plant-growth-promoting rhizobacteria (PGPR), mycorrhizae and soil pathogens, inhabit and form communities.

Of the soil microorganisms, plant-growth-promoting rhizobacteria (PGPR) promote the growth of plants through various enzymatic activities, including fixing atmospheric nitrogen and producing plant growth hormones, and functions to protect plants from various diseases. Also, PGPR produces a quorum-sensing autoinducer, N-acyl homoserine lactones (AHLs), to regulate population density thereof as well as interact with plants, particularly forming a biological membrane in the roots of crops through rhizosphere competence. Through such actions, PGPR is known to be directly involved in resistance induction or growth stimulation of plants. Moreover, PGPR produces plant-growth-stimulating substances, such as gibberellin, auxin, cytokinin, ABA and ethylene, while many studies have been conducted on resistance-associated substances, such as jasmonates, brassinosteroids, salicylic acid, turgorins, strigol, and the like.

### Disclosure

### Technical Problem

Conventional studies and articles associated with *Bacillus amyloliquefaciens* have focused only on the antimicrobial effects thereof, whereas there is no report describing the influence of *B. amyloliquefaciens* on the growth and development of plants or crops. In this regard, the present inventors have conducted intensive and thorough research, which resulted in the remarkable finding that *Bacillus amyloliquefaciens* GYL4, known to produce antibiotic substances, is more useful in plant "growth and development" by a mechanism and a principle completely different from those involved in the antimicrobial effect of the strain. It is therefore an object of the present invention to provide an organic agent for promoting plant growth and an organic agriculture method of promoting plant growth, based on the use of the above-mentioned strain.

### Technical Solution

In order to accomplish the above object, the present invention provides a method including applying a liquid-phase agent for promoting the growth of crops comprising a living bacterium of *Bacillus amyloliquefaciens* GYL4 as an effective component. The *Bacillus amyloliquefaciens* GYL4 strain was deposited at the National Agricultural GeneBank Center in the National Institute of Agricultural Sciences of the Rural Development Administration in South Korea, and was assigned accession number KACC91655P.

The *Bacillus amyloliquefaciens* GYL4 strain, serving as an effective component, inhabits the vicinity of the rhizosphere of crops and additionally, as an endophytic bacterium, migrates into internal plant tissues via roots. Since endophytic bacteria have excellent viability compared to rhizospheric bacteria, the growth-promoting agent for use in the method according to the present invention has sustained action on plants and crops.

The method according to the present invention can be applied to unlimited types of plants as well as crops and fruit trees. Preferably, the agent may effectively enhance the growth and development of crops including bok choy, tomato, cabbage, chili pepper, watermelon, cucumber and zucchini.

The growth-promoting agent for use in the method according to the present invention is prepared by culturing with shaking a living bacterium of *Bacillus amyloliquefaciens* GYL4 at 100 to 200 rpm at 20 to 40°C for 1 to 3 days and mixing the culture thus obtained with water at a volume ratio ranging from 1:0.5 to 1:2.

It is preferred that the strain be cultured with shaking in Tryptic Soy Broth (TSB; MBcell Co., Korea) at 180 rpm at 30°C for 2 days. It is preferred that the culture be obtained by culturing using a fermentor in a 2% TSB medium at 30°C for 2 days. The culture thus obtained may be mixed with water at a volume ratio of 1:0.5 to 1:2, preferably at a volume ratio of 1:1, to give a growth-promoting agent.

The growth-promoting agent prepared as described above was tested for change in cell viability at a high temperature over time and was found to provide stable viable cell counts, and this agent is thus a stable formulation that maintains viable cell counts for up to at least three years (estimated based on one year of shelf life equating to two weeks in a high-temperature test for changes in viability over time).

| Storage Temp. | Repeated No. | Change in viable cell counts (cfu/ml) for B. *amyloliquefaciens* GYL4 at high temperature over time | | | | | | |
|---|---|---|---|---|---|---|---|---|
| | | Week 0 | Week 1 | Week 2 | Week 3 | Week 4 | Week 5 | Week 6 |
| 54°C | 1 | 1.2×10⁹ | 5.4×10⁸ | 4.0×10⁸ | 4.0×10⁸ | 2.7×10⁸ | 2.5×10⁸ | 1.9×10⁸ |
| | 2 | 1.0×10⁹ | 5.0×10⁸ | 4.3×10⁸ | 4.2×10⁸ | 2.5×10⁸ | 2.1×10⁸ | 2.0×10⁸ |
| | 3 | 1.1×10⁹ | 5.8×10⁸ | 4.5×10⁸ | 4.1×10⁸ | 2.8×10⁸ | 2.0×10⁸ | 1.8×10⁸ |

The present invention provides a method of promoting the growth of crops comprising applying the growth-promoting agent to the crops. Spraying of the present agent only once or twice at an early stage of the growth of crops ensures that the bacterial strain effectively inhabits the rhizosphere and migrates inside the crops. It is preferred that the agent be sprayed again 7 to 10 days after first spraying. Also, method according to the present invention can be applied even during the period of raising seedlings, for example, using a watering can to drench crops in the agent to obtain a growth-promoting effect.

### Description of Drawings

FIGS. 1a and 1b show cucumber production yields at indicated dates and cumulative yields respectively, wherein the yield of cucumber cultivated with the growth-promoting agent of the present invention was compared with that in control groups; and
FIG. 2 shows photographs of watermelon for qualitative analysis of watermelon cultivated using the growth-promoting agent of the present invention compared to control groups.

### Mode for Invention

Hereinafter, a detailed description will be given of the growth-promoting agent for use in the method according to the present invention with reference to the appended drawings through the following examples, in which the agent is applied to various crops. The following examples are set forth to illustrate the present invention so as to describe the same in more detail, but are not to be construed as limiting the present invention.

### EXAMPLE 1: (not according to the invention) Preparation of Growth-Promoting Agent

*Bacillus amyloliquefaciens* GYL4 was cultured with shaking in Tryptic Soy Broth (TSB; MBcell Co., Korea) at 180 rpm at 30°C for 2 days. The culture thus obtained was mixed with water at a ratio of 1:1 to give a growth-promoting agent according to the present invention.

As a control, a commercially available growth-promoting agent, EXTN-1 (FarmHannong Co., Ltd, South Korea), was used, which comprises *Bacillus vallismortis* as an effective component. EXTN-1 was diluted with water at a volume ratio of 1:1,000 to give Control 1.

### EXAMPLE 2: Estimation of Growth Promotion and Yield of Cucumber

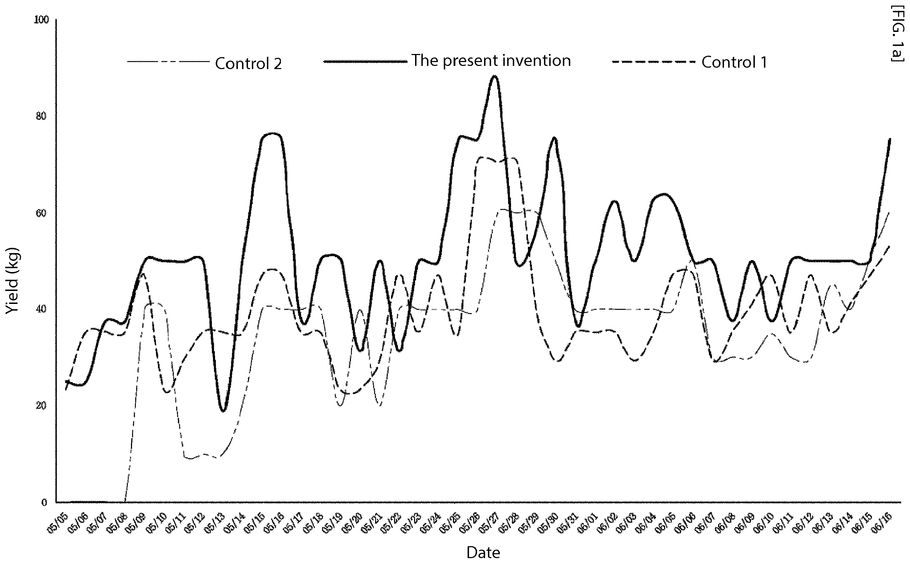

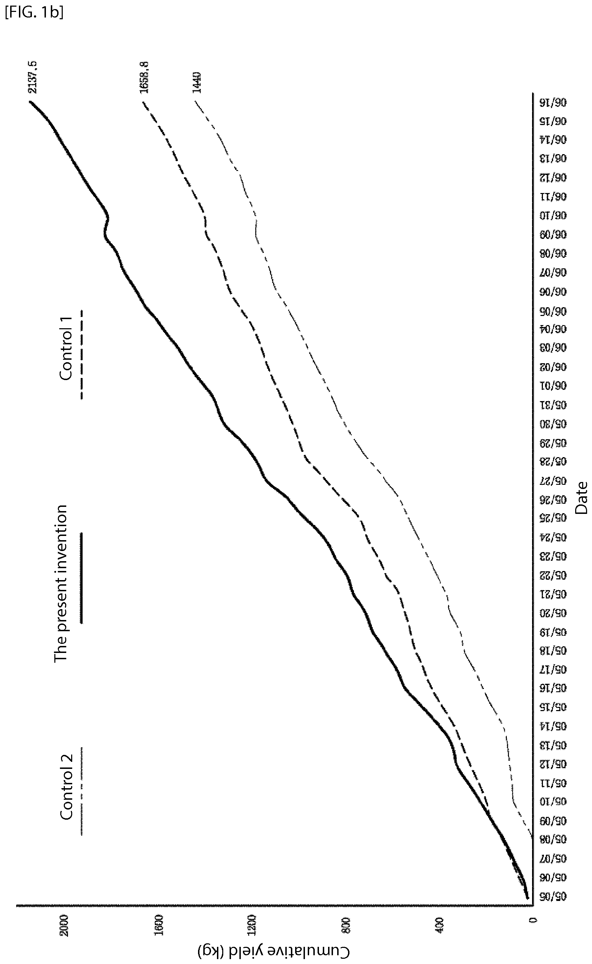

A cucumber variety, Samdong-Chungang, was planted in a greenhouse according to a commonly used method. The growth-promoting agent of the present invention and the growth-promoting agent of Control 1, prepared in the above Example 1, were applied by drenching the soil once at planting and one more time 7 days after planting. Another control group 2 was not treated with any of the growth-promoting agents. In a total land area of 360 m², each experimental plot was 40 m², and each treatment was arranged in a randomized complete block design with three replications. At 60 and 90 days after planting, leaf color and plant height were assessed to estimate the growth of cucumber plants while fruit diameter and fruit weight were assessed to evaluate production yield. The results are given in Table 1, below. FIGS. 1a and 1b show the production yields and cumulative yields of cucumber.

**[TABLE 1]**

| | Leaf color (SPAD) | | Plant height (cm) | | Fruit diameter (cm/plant) | | Fruit weight (g/plant) | | Total yield (May-June) | |
|---|---|---|---|---|---|---|---|---|---|---|
| | Day 60 | Day 90 | Day 60 | Index | Day 60 | Day 90 | Day 60 | Day 90 | Yield (kg/400 plants) | Yield index |
| The present invention | 56.2 | 54.6 | 145.5 | 107 | 27.8 | 31.4 | 188.9 | 201.6 | 2,188 | 149 |
| Control 1 | 52.8 | 50.6 | 141.5 | 104 | 25.2 | 30.6 | 149.2 | 199.9 | 1,700 | 116 |
| Control 2 | 50.2 | 49.6 | 135.8 | 100 | 22.9 | 27.6 | 103.0 | 168.6 | 1, 470 | 100 |

As shown in Table 1, compared to the control groups 1 and 2, the present invention exhibited greater SPAD values of cucumber leaves at both 60 and 90 days after planting and a higher plant height at 60 days, while having a much better effect on fruit diameter and fruit weight at 60 and 90 days after planting. For the period of May to June 2015, the present invention increased the total yield by 49% compared to Control 2 and by 33% compared to Control 1.

Both the present invention and Control 1, compared to Control 2, reached the harvest stage rapidly in early May, but only the present invention maintained cucumber productivity without a yield reduction beyond June, which is the latter half of the harvest season. Collectively, it was found that Control 1 influences plant growth and yield with its rhizosphere microorganism but this microorganism has decreased viability in the rhizosphere as the harvest season goes to the latter part, leading to decreased sustainability in efficacy, whereas the present invention, compared to Control 1, has greater effects on plant growth and yield with its endophytic bacterium and thus has excellent potential for use as an agricultural formulation for soil improvement and crop growth.

### EXAMPLE 3: Estimation of Growth Promotion and Yield of Watermelon

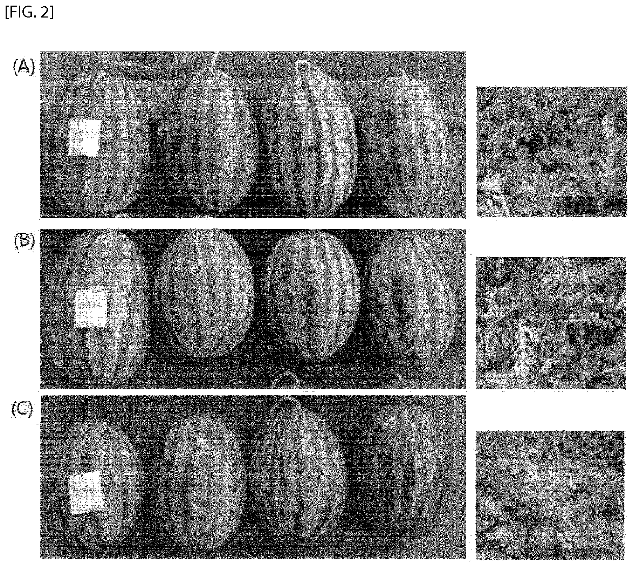

A watermelon variety, SPEED-PLUS, was planted in a greenhouse according to a commonly used method. As described in Example 2, each of the growth-promoting agents was applied to a test group by drenching the soil once at planting and one more time 7 days after planting. Control group 2 was not treated with any of the growth-promoting agents. In a total land area of 450 m², each experimental plot was 50 m², and each treatment was arranged in a randomized complete block design with three replications. At 30, 60 and 92 days after planting, leaf color and plant height were assessed to estimate the growth of watermelon plants while fruit weight and sugar content (Brix) were assessed to evaluate production yield. The results are given in Table 2, below. FIG. 2 shows harvested watermelons and watermelon leaves in the experimental plots receiving treatment according to the present invention (A), Control 1 (B) and Control 2 (C).

**[TABLE 2]**

| | Leaf color (SPAD) | | Plant height (cm) | | Fruit sugar content (Brix/plant) | | Fruit weight (g/plant) | |
|---|---|---|---|---|---|---|---|---|
| | Day 30 | Day 60 | Day 30 | Index | Day 92 | Index | Day 92 | Index |
| The present invention | 50.2 | 54.7 | 58.5 | 102 | 12.1 | 102 | 8.9 | 113 |
| Control 1 | 48.5 | 50.7 | 57.5 | 100 | 11.8 | 100 | 8.3 | 105 |
| Control 2 | 46.3 | 48.2 | 57.5 | 100 | 11.8 | 100 | 7.9 | 100 |

As shown in in Table 2, the growth estimation resulted in the finding that the present invention has much better SPAD values of watermelon leaves than two control groups 1 and 2 at 30 and 60 days. The evaluation of plant height revealed that the early growth was similar in all experimental groups. With respect to yield, the present invention was found to increase fruit weight by 13% compared to Control 2 and by 8% or greater compared to Control 1. In particular, when a watermelon plant was treated with the present invention, watermelon leaves were observed to become thicker, indicating that the present invention may promote greater disease resistance.

### EXAMPLE 4: Estimation of Growth Promotion and Yield of Chili Pepper

A chili pepper variety, Dog-Ya-Cheong-Choeong, was planted in mulched soil in a field according to a commonly used method. The growth-promoting agents as prepared in Example 1 were individually applied by drenching the soil once at planting and one more time 7 days after planting. Control group 2 was not treated with any of the growth-promoting agents. In a total land area of 180 m², each experimental plot was 15 m², and each treatment was arranged in a randomized complete block design with three replications. At 30 and 40 days after planting, leaf color and plant height were assessed to estimate the growth of chili pepper plants. The results are given in Table 3, below.

**[TABLE 3]**

| | Leaf color (SPAD) | | Plant height (cm) | |
|---|---|---|---|---|
| | Day 30 | Day 40 | Day 30 | Day 40 |
| The present invention | 62.8 | 63.2 | 41.4 | 54.1 |
| Control 1 | 63.3 | 62.6 | 40.2 | 54.5 |
| Control 2 | 58.1 | 60.5 | 38.3 | 50.4 |

As shown in Table 3, the evaluation of chili pepper growth resulted in the finding that the present invention provides a higher leaf SPAD value at 40 days compared to two control groups 1 and 2, while plant height was higher in the group treated with the present invention at 30 days compared to the control groups 1 and 2.

### EXAMPLE 5: Estimation of Growth Promotion and Yield of Cabbage

A cabbage variety, YR-Goeul, was planted in mulched soil in a field according to a commonly used method.

As a control group 3, a microbial growth-promoting agent, commercially available under the trade name "Okcheon" (South Korea), was used, which contains *Bacillus amyloliquefaciens* KB3 as an effective component and is used as an organic agriculture material. According to the manufacturer's recommended dosage, Okcheon was diluted 1,000-fold in water and was applied to a test group by drenching the soil.

The growth-promoting agents as prepared in Example 2 and the growth-promoting agent of Control 3 were individually applied to a test group by drenching the soil once at planting and one more time 7 days after planting. Control group 2 was not treated with any one of the growth-promoting agents. In a total land area of 180 m², each experimental plot was 15 m², and each treatment was arranged in a randomized complete block design with three replications. At 20 and 30 days after planting, leaf color and leaf length were assessed to estimate plant growth. 78 days after planting, that is, at the harvest stage, head width and fresh weight were assessed to evaluate production yield. The results are given in Table 4, below.

**[TABLE 4]**

| | Leaf color (SPAD) | | Leaf length (cm) | | Head width (cm) | Fresh weight (kg/plant) |
|---|---|---|---|---|---|---|
| | Day 20 | Day 30 | Day 20 | Day 30 | Day 78 | Day 78 |
| The present invention | 49.3 | 54.0 | 19.8 | 33.0 | 20.8 | 1.8 |
| Control 1 | 50.0 | 50.9 | 19.0 | 30.5 | 19.5 | 1.5 |
| Control 3 | 52.1 | 52.4 | 18.3 | 29.8 | 20.8 | 1.8 |
| Control 2 | 48.3 | 53.0 | 16.5 | 27.8 | 20.2 | 1.6 |

As shown in Table 4, compared to the control groups 1, 2 and 3, the present invention was found to confer a much better leaf SPAD value at 30 days as well as have an excellent effect on leaf length, indicating that the present invention has a growth-enhancing effect superior to the control groups. Also, with regard to cabbage yield and quality, the present invention was observed to have excellent effects on head width and fresh weight compared to the control groups 1 and 2.

### EXAMPLE 6: Estimation of Growth of Tomato

A tomato variety, Pink-Giant, was cultivated in mulched soil in a greenhouse. The growth-promoting agents used in Example 5 were individually applied by drenching the soil once at planting and one more time at 14 days after planting. Control group 2 was not treated with any of the growth-promoting agents. At 21 days after planting, the fresh weight of the tomatoes was 30.1 g in the present invention group, and was 23.8 g, 21.0 g and 24.7 g in the control groups 1, 2 and 3, respectively. The present invention was found to be most effective on the early growth of tomatoes compared to the control groups.

### EXAMPLE 7: Estimation of Growth of bok choy

A bok choy variety, Chingen-Sai, was cultivated in mulched soil in a greenhouse. The growth-promoting agents as used in Example 5 were individually applied by drenching the soil once at planting and one more time at 14 days after planting. Control group 2 was not treated with any of the growth-promoting agents. At 21 days after planting, the fresh weight of the bok choy was 178.6 g in the present invention group, and was 170.8 g, 142.6 g and 178.8 g in the control groups 1, 2 and 3, respectively. The present invention was found to have a better effect on the early growth of bok choy compared to the control groups 1, 2 and 3.

## Claims

1. A method of promoting growth of a crop, comprising:
mixing a liquid-phase growth-promoting agent for a crop with water; and
applying a mixture thus obtained to the crop;
wherein said liquid-phase growth-promoting agent comprises a living bacterium of *Bacillus amyloliquefaciens* GYL4 (accession number KACC91655P) as an effective component.

2. The method of claim 1, wherein the crop is selected from among bok choy, tomato, cabbage, chili pepper, watermelon, cucumber and zucchini.

## Patentansprüche

1. Ein Verfahren zur Förderung des Wachstums einer Nutzpflanze, das Folgendes umfasst: Mischung eines Wachstumsförderers der Flüssigphase einer Kultur mit Wasser; und Aufbringen einer so gewonnenen Mischung auf die Pflanze, wobei das Wachstumsförderungsmittel der Flüssigphase ein lebendes Bakterium von Bacillus amyloliquefaciens GYL4 (Zugangsnummer KACC91655P) als wirksame Komponente enthält.

2. Verfahren nach Anspruch 1, **dadurch gekennzeichnet, dass** die Nutzpflanze ausgewählt ist aus Pak Choy (Senfkohl), Tomaten, Kohl, Chili-Pfeffer, Wassermelone, Gurke und Zucchini.

## Revendications

1. Procédé pour favoriser la croissance d'une culture, comprenant: le mélange d'un agent favorisant la croissance en phase liquide pour une culture avec de l'eau; et Application d'un mélange ainsi obtenu sur la culture, dans laquelle ledit agent favorisant la croissance en phase liquide comprend une bactérie vivante de Bacillus amyloliquefaciens GYL4 (numéro d'accès KACC91655P) en tant qu'agent efficace.

2. Procédé selon la revendication 1, dans lequel la culture est choisie parmi le bok choy, la tomate, le chou, le piment, la pastèque, concombre et courgettes.
